# DEMANDE DE BREVET EUROPEEN

(11) **EP 3 248 558 A1**
(43) Date de publication de la demande: **29.11.2017**
(21) Numéro de dépôt: 17171757.2
(22) Date de dépôt: 18.05.2017
(51) Int. Cl.: A61B 17/72, A61F 2/42, A61F 2/30

(54) **IMPLANT D'ARTHRODÈSE INTERPHALANGIENNE**

(30) Priorité: 19.05.2016 FR 1654466
(71) Demandeur: Fournitures Hospitalieres Industrie, 29000 Quimper (FR); FH Orthopedics, 68990 Heimsbrunn (FR); Roussignol, Xavier, 76250 Deville les Rouen (FR); Polle, Gerard, 76230 Bois-Guillaume (FR); Laborde, Julien, 31400 Toulouse (FR); Etchervers, Jean-Pierre, 64310 Ascain (FR); Darcel, Veronique, 33000 Bordeaux (FR); Cordier, Guillaume, 33640 Ayguemorte les Graves (FR); Sevestre, Francois-Xavier, 35700 Rennes (FR); De Rouvray, Thibault, 37540 Saint Cyr Sur Loire (FR)
(72) Inventeur: ROUSSIGNOL, Xavier, 76250 Deville les Rouen (FR); POLLE, Gérard, 76230 Bois-Guillaume (FR); LABORDE, Julien, 31400 Toulouse (FR); ETCHEVERS, Jean-Pierre, 64310 Ascain (FR); DARCEL, Véronique, 33000 Bordeaux (FR); CORDIER, Guillaume, 33640 Ayguemorte les Graves (FR); SEVESTRE, François-Xavier, 35700 Rennes (FR); DE ROUVRAY, Thibault, 37540 Saint Cyr sur Loire (FR); ROUYER, Guillaume, 29000 Quimper (FR)
(74) Mandataire: Verriest, Philippe

(57) **Abrégé**

Cet implant d'arthrodèse interphalangienne (2) comprend une partie d'ancrage proximale (6) destinée à être engagée et ancrée dans le canal médullaire d'une première phalange sectionnée selon un premier plan d'ostéotomie, la partie d'ancrage proximale (6) comportant une pluralité de branches d'ancrage proximales (12) pourvues chacune d'au moins un élément d'ancrage osseux (13) ; et une partie d'ancrage distale (8), reliée à la partie d'ancrage proximale (6), destinée à être engagée et ancrée dans le canal médullaire d'une deuxième phalange sectionnée selon un deuxième plan d'ostéotomie, la partie d'ancrage distale (8) comportant une pluralité de branches d'ancrage distales (18) pourvues chacune d'au moins un élément d'ancrage osseux (19). La partie d'ancrage distale (8) comprend une portion de butée (16) comportant une surface de butée (17) destinée à prendre appui contre une surface sectionnée de la première phalange s'étendant selon le premier plan d'ostéotomie.

## Description

La présente invention concerne un implant d'arthrodèse interphalangienne destiné à permettre une fusion osseuse entre une première et une deuxième phalanges d'une articulation interphalangienne, telle qu'une articulation interphalangienne d'un pied, et notamment une articulation interphalangienne proximale d'un pied.

Il arrive qu'un pied présentant un hallux valgus, c'est-à-dire une déformation correspondant à une déviation du premier métatarsien en varus et du gros orteil en valgus, présente également une déformation du deuxième orteil, voire du troisième orteil. En effet, sous l'influence du gros orteil, le deuxième orteil, et éventuellement le troisième orteil, peuvent se déformer et prendre une forme de griffe.

Certaines déformations des deuxième et troisième orteils nécessitent une intervention chirurgicale destinée à redonner un aspect normal au pied, et à supprimer la douleur induite par ces déformations. Une telle intervention chirurgicale peut consister en une arthrodèse interphalangienne entre les première et deuxième phalanges du deuxième orteil, et éventuellement en une arthrodèse interphalangienne entre les première et deuxième phalanges du troisième orteil, de manière à immobiliser la ou les articulations concernées afin de supprimer la douleur du patient.

Une arthrodèse interphalangienne consiste généralement à :
- effectuer une ostéotomie des première et deuxième phalanges de part et d'autre de l'articulation concernée et selon un premier et un deuxième plans d'ostéotomie s'étendant respectivement perpendiculairement aux première et deuxième phalanges,
- prévoir un implant d'arthrodèse interphalangienne comprenant une partie d'ancrage proximale, et une partie d'ancrage distale reliée à la partie d'ancrage proximale,
- usiner le canal médullaire de la première phalange et le canal médullaire de la deuxième phalange de telle sorte qu'ils présentent des dimensions correspondant sensiblement aux dimensions extérieures des parties d'ancrage proximale et distale,
- insérer et ancrer la partie d'ancrage proximale dans le canal médullaire de la première phalange préalablement sectionnée selon le premier plan d'ostéotomie, et
- insérer et ancrer la partie d'ancrage distale dans le canal médullaire de la deuxième phalange préalablement sectionnée selon le deuxième plan d'ostéotomie, de telle sorte que les surfaces sectionnées des première et deuxième phalanges soient sensiblement en appui l'une contre l'autre.

De façon connue, les parties d'ancrage proximale et distale de l'implant d'arthrodèse interphalangienne peuvent comporter respectivement une pluralité de branches d'ancrage proximales pourvues chacune d'au moins un élément d'ancrage osseux, et une pluralité de branches d'ancrage distales pourvues chacune d'au moins un élément d'ancrage osseux.

Cependant, une telle configuration de l'implant d'arthrodèse interphalangienne peut induire, lors de l'insertion de la partie d'ancrage distale dans le canal médullaire de la deuxième phalange, un déplacement de la partie d'ancrage proximale par rapport à la première phalange, et plus particulièrement un enfoncement de la partie d'ancrage proximale dans le canal médullaire de la première phalange au-delà de sa position prédéterminée. Un tel enfoncement de l'implant peut nuire à l'ancrage final de la partie d'ancrage distale dans la deuxième phalange, et donc requérir une éventuelle reprise ultérieure de l'implant d'arthrodèse interphalangienne par le chirurgien.

La présente invention vise à remédier à cet inconvénient.

Le problème technique à la base de l'invention consiste donc à fournir un implant d'arthrodèse interphalangienne qui assure une fixation aisée et fiable de deux phalanges sectionnées d'une articulation interphalangienne.

A cet effet, la présente invention concerne un implant d'arthrodèse interphalangienne destiné à permettre une fusion osseuse entre une première et une deuxième phalanges d'une articulation interphalangienne, l'implant d'arthrodèse interphalangienne comprenant :
- une partie d'ancrage proximale destinée à être engagée et ancrée dans le canal médullaire de la première phalange préalablement sectionnée selon un premier plan d'ostéotomie, la partie d'ancrage proximale comportant une pluralité de branches d'ancrage proximales pourvues chacune d'au moins un élément d'ancrage osseux,
- une partie d'ancrage distale, reliée à la partie d'ancrage proximale, destinée à être engagée et ancrée dans le canal médullaire de la deuxième phalange préalablement sectionnée selon un deuxième plan d'ostéotomie, la partie d'ancrage distale comportant une pluralité de branches d'ancrage distales pourvues chacune d'au moins un élément d'ancrage osseux, la partie d'ancrage distale comprenant une portion de butée comportant une surface de butée destinée à prendre appui contre une surface sectionnée de la première phalange s'étendant selon le premier plan d'ostéotomie,

caractérisé ce que chaque branche d'ancrage proximale comporte une première extrémité et une deuxième extrémité opposée à la première extrémité respective, la première extrémité de chaque branche d'ancrage proximale étant plus proche de la partie d'ancrage distale que la deuxième extrémité respective, et chaque branche d'ancrage distale comporte une première extrémité et une deuxième extrémité opposée à la première extrémité respective, la première extrémité de chaque branche d'ancrage distale étant plus proche de la partie d'ancrage proximale que la deuxième extrémité respective, et en ce que la partie d'ancrage proximale comporte une portion d'extrémité proximale reliant les deuxièmes extrémités des branches d'ancrage proximales, et la partie d'ancrage distale comporte une portion d'extrémité distale reliant les deuxièmes extrémités des branches d'ancrage distales.

Une telle configuration de l'implant d'arthrodèse interphalangienne, et plus particulièrement la présence de la surface d'appui, permet de limiter la profondeur d'enfoncement de la partie d'ancrage proximale dans le canal médullaire de la phalange respective, et donc d'éviter un ancrage ultérieur inapproprié de la partie d'ancrage distale sur la phalange respective. Ces dispositions permettent ainsi d'assurer une fixation aisée et fiable de deux phalanges d'une articulation interphalangienne.

Il convient d'être noté que les branches d'ancrage distales et proximales confèrent à l'implant une certaine élasticité garantissant un ancrage optimal des parties d'ancrages distales et proximales dans les canaux médullaires respectifs. De plus, la présence d'éléments d'ancrage sur les branches d'ancrage distales et proximales augmente la stabilité de l'implant (notamment en rotation et en translation), et donc assure une consolidation osseuse rapide et optimale.

L'implant d'arthrodèse interphalangienne peut en outre présenter une ou plusieurs des caractéristiques suivantes, prises seules ou en combinaison.

Selon un mode de réalisation de l'invention, la partie d'ancrage proximale s'étend globalement selon un premier axe d'extension, et la partie d'ancrage distale s'étend globalement selon un deuxième axe d'extension.

Selon un mode de réalisation de l'invention, les premier et deuxième axes d'extension étant inclinés l'un par rapport à l'autre. Les premier et deuxième axes d'extension peuvent par exemple être inclinés l'un par rapport à l'autre d'un angle compris entre 3 et 15°.

Selon un mode de réalisation de l'invention, les branches d'ancrage proximales sont réparties, avantageusement régulièrement réparties, autour du premier axe d'extension, et les branches d'ancrage distales sont réparties, avantageusement régulièrement réparties, autour du deuxième axe d'extension.

Selon un mode de réalisation de l'invention, la surface de butée est sensiblement plane.

Selon un mode de réalisation de l'invention, la surface de butée s'étend de manière inclinée par rapport à l'axe d'extension de la partie d'ancrage proximale.

Selon un mode de réalisation de l'invention, la surface de butée est annulaire, et peut par exemple s'étendre coaxialement avec le deuxième axe d'extension.

Selon un mode de réalisation de l'invention, la surface de butée est formée par un épaulement, et par exemple par un épaulement annulaire.

Selon un mode de réalisation de l'invention, la partie d'ancrage proximale comporte en outre une portion de liaison reliant la portion de butée et les branches d'ancrage proximales.

Selon un mode de réalisation de l'invention, la portion de liaison présente une surface extérieure sensiblement lisse.

Selon un mode de réalisation de l'invention, la portion de liaison présente une section transversale sensiblement circulaire.

Selon un mode de réalisation de l'invention, les premières extrémités des branches d'ancrages proximales sont reliées à la portion de liaison, et les premières extrémités des branches d'ancrages distales sont reliées à la portion de butée.

Selon un mode de réalisation de l'invention, les branches d'ancrages proximales s'étendent à partir de la portion de liaison, et les branches d'ancrages distales s'étendent à partir de la portion de butée.

Selon un mode de réalisation de l'invention, les branches d'ancrage proximales et distales ont chacune une forme globalement allongée, et dans lequel les branches d'ancrage proximales convergent sensiblement en direction de la portion d'extrémité proximale et les branches d'ancrage distales convergent sensiblement en direction de la portion d'extrémité distale.

Selon un mode de réalisation de l'invention, les portions d'extrémité proximale et distale présente chacune une forme générale d'ogive.

Selon un mode de réalisation de l'invention, l'implant d'arthrodèse interphalangienne est monobloc.

Selon un mode de réalisation de l'invention, chaque branche d'ancrage proximale et distale comporte une pluralité d'éléments d'ancrage disposés le long de la branche d'ancrage respective.

Selon un mode de réalisation de l'invention, chaque élément d'ancrage est un cran d'ancrage.

Selon un mode de réalisation de l'invention, l'implant d'arthrodèse interphalangienne est radiotransparent.

Selon un mode de réalisation de l'invention, l'implant d'arthrodèse interphalangienne est en matière plastique, tel qu'en polymère.

Selon un mode de réalisation de l'invention, l'implant d'arthrodèse interphalangienne est en PEEK.

Selon un mode de réalisation de l'invention, la portion d'extrémité proximale comporte une face d'extrémité proximale sensiblement plane et s'étendant transversalement, et par exemple perpendiculairement, au premier axe d'extension, et la portion d'extrémité distale comporte une face d'extrémité distale sensiblement plane et s'étendant transversalement, et par exemple perpendiculairement, au deuxième axe d'extension.

De toute façon l'invention sera bien comprise à l'aide de la description qui suit en référence au dessin schématique annexé représentant, à titre d'exemple non limitatif, une forme d'exécution de cet implant d'arthrodèse interphalangienne.
Figure 1 est une vue en perspective d'un implant d'arthrodèse interphalangienne selon la présente invention.
Figures 2 et 3 sont des vues respectivement de côté et de dessus de l'implant d'arthrodèse interphalangienne de la figure 1.
Figure 4 est une vue de côté d'un deuxième orteil d'un pied schématisant des premier et deuxième plans d'ostéotomie respectivement des première et deuxième phalanges de l'articulation interphalangienne proximale du deuxième orteil.
Figures 5 et 6 sont des vues montrant des étapes d'usinages des canaux médullaires des première et deuxième phalanges de la figure 4.
Figures 7 à 9 sont des vues montrant différentes étapes de fixation de l'implant d'arthrodèse interphalangienne de la figure 1 dans les première et deuxième phalanges de la figure 4.
Figure 10 est une vue en perspective d'une gamme d'implants d'arthrodèse interphalangienne selon la présente invention.

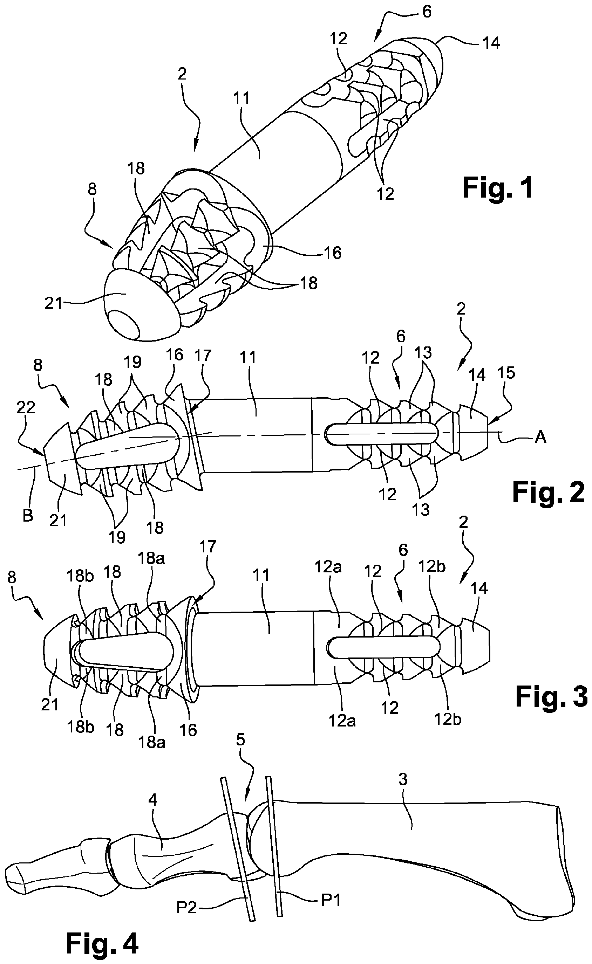

Les figures 1 à 3 représentent un implant d'arthrodèse interphalangienne 2 destiné à permettre une fusion osseuse entre une première et une deuxième phalanges 3, 4 d'une articulation interphalangienne proximale 5 d'un pied. L'implant d'arthrodèse interphalangienne 2 est avantageusement monobloc, et peut être réalisé par exemple en matière plastique, telle qu'en PEEK.

L'implant d'arthrodèse interphalangienne 2 comprend une partie d'ancrage proximale 6 destinée à être engagée et ancrée dans le canal médullaire 7 de la première phalange 3 préalablement sectionnée selon un premier plan d'ostéotomie P1, et une partie d'ancrage distale 8 destinée à être engagée et ancrée dans le canal médullaire 9 de la deuxième phalange 4 préalablement sectionnée selon un deuxième plan d'ostéotomie P2.

La partie d'ancrage proximale 6 est de forme globalement allongée, et s'étend globalement selon un premier axe d'extension A. La partie d'ancrage proximale 6 comporte une portion de liaison 11 reliée à la partie d'ancrage distale 8 et s'étendant selon le premier axe d'extension A. Selon le mode de réalisation représenté sur les figures, la portion de liaison 11 présente une surface extérieure lisse et une section transversale circulaire.

La partie d'ancrage proximale 6 comporte en outre une pluralité de branches d'ancrage proximales 12, par exemple quatre, régulièrement réparties autour du premier axe d'extension A. Chaque branche d'ancrage proximale 12 présente une forme globalement allongée, et comporte une première extrémité 12a reliée à la portion de liaison 11, et une deuxième extrémité 12b opposée à la première extrémité 12a respective. Ainsi, chaque branche d'ancrages proximale 12 s'étend à partir de la portion de liaison 11.

Chaque branche d'ancrage proximale 12 est pourvue avantageusement d'une pluralité de crans d'ancrage osseux 13 disposés le long de la branche d'ancrage proximale 12 respective.

La partie d'ancrage proximale 6 comporte également une portion d'extrémité proximale 14 reliant les deuxièmes extrémités 12b des branches d'ancrage proximales 12. Selon le mode de réalisation représenté sur les figures, la portion d'extrémité proximale 14 présente une forme générale d'ogive, et comporte une face d'extrémité proximale 15 plane et s'étendant perpendiculairement au premier axe d'extension A.

La partie d'ancrage distale 8 est de forme globalement allongée, et s'étend globalement selon un deuxième axe d'extension B incliné par rapport au premier axe d'extension A d'un angle compris par exemple entre 3 et 15°.

La partie d'ancrage distale 8 comporte une portion de butée 16 comportant une surface de butée 17 destinée à prendre appui contre une surface sectionnée de la première phalange 3. Il doit être noté que la portion de liaison 11 est configurée pour relier la portion de butée 16 et les branches d'ancrage proximales 12.

Selon le mode de réalisation représenté sur les figures, la surface de butée 17 est formée par un épaulement configuré de telle sorte que la portion de butée 16 présente un diamètre extérieur supérieur au diamètre extérieur de la portion de liaison 11. Avantageusement, la surface de butée 17 est annulaire et s'étend coaxialement avec le deuxième axe d'extension. Selon le mode de réalisation représenté sur les figures, la surface de butée 17 est plane et s'étend de manière inclinée par rapport au premier axe d'extension A.

La partie d'ancrage distale 8 comporte en outre une pluralité de branches d'ancrage distales 18, par exemple quatre, régulièrement réparties autour du deuxième axe d'extension B. Chaque branche d'ancrage distale 18 présente une forme globalement allongée, et comporte une première extrémité 18a reliée à la portion de butée 16, et une deuxième extrémité 18b opposée à la première extrémité 18a respective. Ainsi, chaque branche d'ancrages distale 18 s'étend à partir de la portion de butée 16.

Chaque branche d'ancrage distale 18 est pourvue avantageusement d'une pluralité de crans d'ancrage osseux 19 disposés le long de la branche d'ancrage distale 18 respective.

La partie d'ancrage distale 8 comporte également une portion d'extrémité distale 21 reliant les deuxièmes extrémités 18b des branches d'ancrage distales 18. Selon le mode de réalisation représenté sur les figures, la portion d'extrémité distale 21 présente une forme générale d'ogive, et comporte une face d'extrémité distale 22 plane et s'étendant perpendiculairement au deuxième axe d'extension B.

Selon le mode de réalisation représenté sur les figures, les branches d'ancrage proximales 12 convergent sensiblement en direction de la portion d'extrémité distale 14, et les branches d'ancrage distales 18 convergent sensiblement en direction de la portion d'extrémité distale 21.

Un procédé de correction d'une déformation du deuxième orteil d'un patient atteint d'un hallux valgus à l'aide d'un implant d'arthrodèse interphalangienne 2 selon la présente invention va maintenant être décrit en se référant plus particulièrement aux figures 4 à 9.

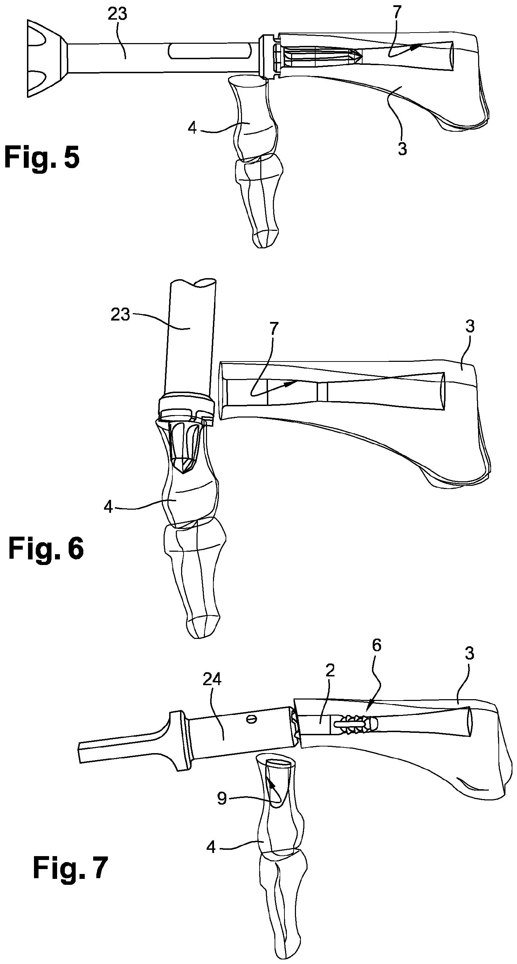

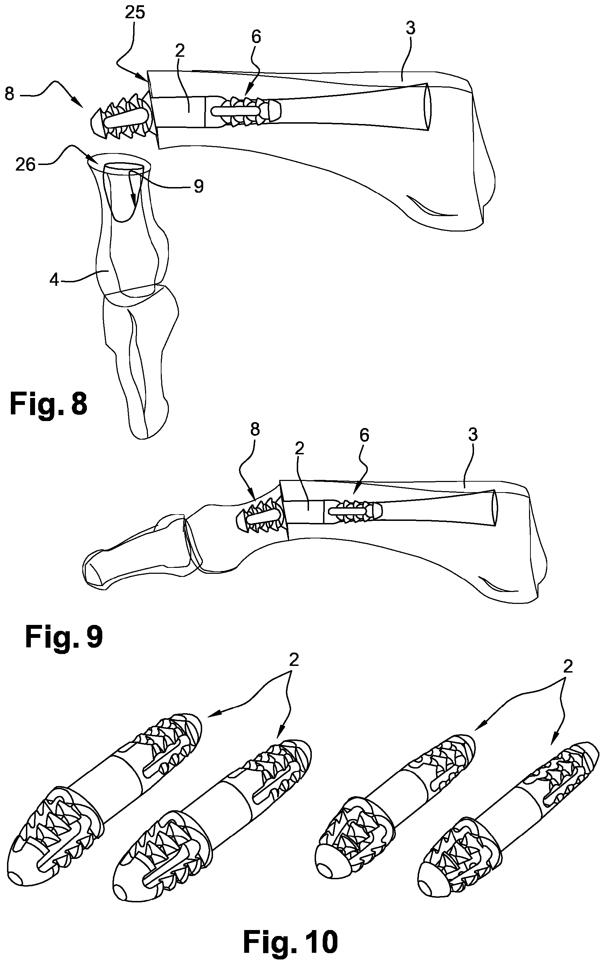

Un tel procédé de correction comprend les étapes consistant à :
- effectuer une ostéotomie des première et deuxième phalanges 3, 4 du deuxième orteil de part et d'autre de l'articulation interphalangienne proximale et selon un premier et un deuxième plans d'ostéotomie P1, P2 s'étendant respectivement sensiblement perpendiculairement aux première et deuxième phalanges 3, 4 (voir la figure 4),
- usiner, à l'aide d'un outil de conformation 23, le canal médullaire 7 de la première phalange 3 et le canal médullaire 9 de la deuxième phalange 4 de telle sorte qu'ils présentent des dimensions correspondant sensiblement aux dimensions extérieures des parties d'ancrage proximale et distale 6, 8 de l'implant d'arthrodèse interphalangienne 2 (voir les figures 5 et 6),
- insérer et ancrer, à l'aide d'un outil de préhension 24, la partie d'ancrage proximale 6 dans le canal médullaire 7 de la première phalange 3 préalablement sectionnée selon le premier plan d'ostéotomie P1, de telle sorte que la surface de butée 17 prenne appui contre la surface sectionnée 25 de la première phalange 3 s'étendant selon le premier plan d'ostéotomie P1 (voir les figures 7 et 8),
- insérer et ancrer la partie d'ancrage distale 8 dans le canal médullaire 9 de la deuxième phalange 4 préalablement sectionnée selon le deuxième plan d'ostéotomie P2, de telle sorte que les surfaces sectionnées 25, 26 des première et deuxième phalanges 3, 4 soient en appui l'une contre l'autre.

Comme montré sur la figure 10, l'implant d'arthrodèse interphalangienne 2 peut être décliné par exemple en deux angulations, c'est-à-dire en deux valeurs de l'angle d'inclinaison entre les premier et deuxième axes d'extension A, B, et en deux tailles. Une telle déclinaison de l'implant d'arthrodèse interphalangienne 2 permet de former une gamme d'implants comprenant quatre implants différents. Toutefois, l'implant d'arthrodèse interphalangienne 2 pourrait être décliné en plus de deux angulations, par exemple en trois ou quatre angulations différentes, et en plus de deux tailles, par exemple en trois ou quatre tailles différents, afin de répondre aux différentes anatomies qui peuvent être rencontrées.

Comme il va de soi, l'invention ne se limite pas à la seule forme d'exécution de cet implant d'arthrodèse interphalangienne, décrite ci-dessus à titre d'exemple, elle en embrasse au contraire toutes les variantes de réalisation.

## Revendications

1. Implant d'arthrodèse interphalangienne (2) destiné à permettre une fusion osseuse entre une première et une deuxième phalanges (3, 4) d'une articulation interphalangienne, l'implant d'arthrodèse interphalangienne comprenant :
- une partie d'ancrage proximale (6) destinée à être engagée et ancrée dans le canal médullaire (7) de la première phalange (3) préalablement sectionnée selon un premier plan d'ostéotomie (P1), la partie d'ancrage proximale (6) comportant une pluralité de branches d'ancrage proximales (12) pourvues chacune d'au moins un élément d'ancrage osseux (13),
- une partie d'ancrage distale (8), reliée à la partie d'ancrage proximale (6), destinée à être engagée et ancrée dans le canal médullaire (9) de la deuxième phalange (4) préalablement sectionnée selon un deuxième plan d'ostéotomie (P2), la partie d'ancrage distale (8) comportant une pluralité de branches d'ancrage distales (18) pourvues chacune d'au moins un élément d'ancrage osseux (19), la partie d'ancrage distale (8) comprenant une portion de butée (16) comportant une surface de butée (17) destinée à prendre appui contre une surface sectionnée (25) de la première phalange (3) s'étendant selon le premier plan d'ostéotomie (P1),
**caractérisé en ce que** chaque branche d'ancrage proximale (12) comporte une première extrémité (12a) et une deuxième extrémité (12b) opposée à la première extrémité (12a) respective, la première extrémité (12a) de chaque branche d'ancrage proximale (12) étant plus proche de la partie d'ancrage distale (8) que la deuxième extrémité (12b) respective, et chaque branche d'ancrage distale (18) comporte une première extrémité (18a) et une deuxième extrémité (18b) opposée à la première extrémité (18a) respective, la première extrémité (18a) de chaque branche d'ancrage distale (18) étant plus proche de la partie d'ancrage proximale (6) que la deuxième extrémité (18b) respective, et **en ce que** la partie d'ancrage proximale (6) comporte une portion d'extrémité proximale (14) reliant les deuxièmes extrémités (12b) des branches d'ancrage proximales (12), et la partie d'ancrage distale (8) comporte une portion d'extrémité distale (21) reliant les deuxièmes extrémités (18b) des branches d'ancrage distales (18).

2. Implant d'arthrodèse interphalangienne selon la revendication 1, dans lequel la surface de butée (17) est sensiblement plane.

3. Implant d'arthrodèse interphalangienne selon la revendication 1 ou 2, dans lequel la surface de butée (17) est annulaire.

4. Implant d'arthrodèse interphalangienne selon l'une quelconque des revendications 1 à 3, dans lequel la partie d'ancrage proximale (6) s'étend globalement selon un premier axe d'extension (A), et la partie d'ancrage distale (8) s'étend globalement selon un deuxième axe d'extension (B).

5. Implant d'arthrodèse interphalangienne selon la revendication 4, dans lequel les premier et deuxième axes d'extension (A, B) sont inclinés l'un par rapport à l'autre.

6. Implant d'arthrodèse interphalangienne selon la revendication 4 ou 5, dans lequel les branches d'ancrage proximales (12) sont réparties autour du premier axe d'extension (A), et les branches d'ancrage distales (18) sont réparties autour du deuxième axe d'extension (B).

7. Implant d'arthrodèse interphalangienne selon l'une quelconque des revendications 1 à 6, dans lequel la partie d'ancrage proximale (6) comporte en outre une portion de liaison (11) reliant la portion de butée (16) et les branches d'ancrage proximales (12).

8. Implant d'arthrodèse interphalangienne selon la revendication 7, dans lequel la portion de liaison (11) présente une surface extérieure sensiblement lisse.

9. Implant d'arthrodèse interphalangienne selon la revendication 7 ou 8, dans lequel la portion de liaison (11) présente une section transversale sensiblement circulaire.

10. Implant d'arthrodèse interphalangienne selon l'une quelconque des revendications 7 à 9, dans lequel les premières extrémités (12a) des branches d'ancrages proximales (12) sont reliées à la portion de liaison (11), et les premières extrémités (18a) des branches d'ancrages distales (18) sont reliées à la portion de butée (16).

11. Implant d'arthrodèse interphalangienne selon l'une quelconque des revendications 1 à 10, dans lequel les branches d'ancrage proximales et distales (12, 18) ont chacune une forme globalement allongée, et dans lequel les branches d'ancrage proximales (12) convergent sensiblement en direction de la portion d'extrémité proximale (14) et les branches d'ancrage distales (18) convergent sensiblement en direction de la portion d'extrémité distale (21).

12. Implant d'arthrodèse interphalangienne selon l'une quelconque des revendications 1 à 11, dans lequel les portions d'extrémité proximale et distale (14, 21) présente chacune une forme générale d'ogive.

13. Implant d'arthrodèse interphalangienne selon l'une quelconque des revendications 1 à 12, lequel est monobloc.
